# EUROPEAN PATENT SPECIFICATION

(11) **EP 0 654 659 B1**
(45) Date of publication and mention of the grant of the patent: **12.02.1997**
(21) Application number: 95101097.4
(22) Date of filing: 08.06.1989
(51) Int. Cl.: G01N 33/52, G01N 33/543

(54) **Defined volume test device**
Testvorrichtung mit definiertem Volumen
Dispositif d'épreuve de volume déterminé

(30) Priority: 09.06.1988 US 205230; 03.05.1989 US 347547
(43) Date of publication of application: 24.05.1995
(62) Divisional of application: 89110393.9
(73) Proprietor: BOEHRINGER MANNHEIM CORPORATION, Indianapolis, Indiana 46250-0528 (US)
(72) Inventor: Daffern, George M., Sunnyvale, CA 94087 (US); Thompson, Tracy N., Palo Alto, CA 94303 (US)
(74) Representative: Jung, Michael, Dr.

(56) References cited:
- EP-A- 0 071 169
- EP-A- 0 154 839

## Description

The invention relates to an improved, disposable device for chemical analysis of liquids. More particularly, it concerns multilayer elements for determining the concentration of an analyte in a liquid.

Multilayer test elements have been extensively used in laboratory environments to detect an analyte of interest. Semi-quantitative or qualitative analyses which do not involve sample size control, such as pregnancy tests, have been successfully adapted for use in the home or doctor's office. However, adapting quantitative tests for use by personnel without laboratory skills or without relatively expensive equipment has proven difficult. Unfortunately, detection of certain analytes such as glucose, alcohol or cholesterol requires quantitative tests which need to be relatively accurate to be useful.

One difficulty in adapting quantitative tests has been accurate dosing of the test sample into or onto the assay device. One method of avoiding the need for control of sample size is performing analyses that determine the reaction rate rather than determining the amount of the analyte in the sample. Since reaction rates change with temperature and a number of other variables, simultaneous evaluation of controls has frequently been required. Additionally, two or more readings are required to calculate a rate.

For assay of analytes such as glucose, cholesterol and alcohol, a blood sample is the preferred sample. To avoid processing the blood to obtain serum or plasma, many tests have been performed on whole blood. A particular problem encountered in quantitation of analytes in whole blood is the presence of red blood cells which causes interference in the detection of color changes of dyes that absorb in the same wavelength range as hemoglobin. Cellular components in blood have been blocked or filtered from the determining layer by a number of methods including use of cellulose, amino acids, glass fibers, or carbohydrate. Fluid metering with the concurrent removal of cellular components of blood has also been used.

Additionally, barrier or blocking layers have been used to segregate cellular components from the serum portion of a whole blood sample. Such barriers used in prior art elements generally are required to be permeable to the ligand, the reagents of the reagent matrix layer, or products of their interaction. In such devices, determination of the detectable species is made from the detecting layer surface or determination surface of the reagent matrix layer on the opposite side of the reagent matrix layer from the dosing surface. Alternatively, a barrier may be included on the element to confine an applied sample to a predetermined region of the element surface.

There is a need for a device that is easy to operate and accurately measure analytes such as glucose, cholesterol and alcohol in whole blood.

Multilayer reagent strips are shown in U.S. Patent 3,672,845. U.S. Patents 3,993,451 and 4,438,067 disclose the use of particulate reagents, and U.S. Patent 3,723,064 discloses the use of a membrane having regions of differing permeability. U.S. Patent 3,783,105 demonstrates the use of freeze-dried reagents. Other multilayer test designs are shown by U.S. Patents 3,980,437, 4,042,335, 4,144,306, 4,160,008, 4,178,153, 4,292,272, 4,318,985, 4,387,990, 4,427,632, 4,452,887, 4,532,107, 4,604,264, and 4,668,472. Another alternative to the multilayer test device approach is shown in U.S. Patent 3,476,515, which discloses rupturable sample and reagent pouches. U.S. Patent 3,158,532 teaches the manufacture of a filter which has pores of graduated size and filtration capacity.

Specific reagents have been found useful in assays for the presence of glucose, cholesterol, alcohol, and other analytes (see, for example, U.S. Patents 2,912,309 and 2,981,606). Buffers, stabilizers, dyes, and other detectable moieties have been incorporated into multilayer devices and test strips.

Cellular components in blood have been blocked or filtered from the reagent matrix layer by use of cellulose (U.S. Patent 3,092,465), amino acids (U.S. Patent 3,552,928), glass fibers (U.S. Patent 4,477,575), and carbohydrate (U.S. Patent 4,678,757). Fluid metering with the concurrent removal of cellular components of blood is addressed in U.S. Patents 4,250,257 and 4,260,392.

Barrier layers in multilayer elements are shown in U.S. Patents 3,992,158, 4,166,093, 4,255,384, 4,256,693, 4,363,874, 4,390,343, 4,478,944. 4,631,174, and 4,066,403 (Reissue patent 30,267).

U.S. Patent No. 4,552,925 describes a whole blood separation method and test using same. The method separates whole blood into a substantially colorless fluid and the red cell component.

U.S. Patent No. 4,774,192 describes a dry chemistry reagent system for detection of analytes such as glucose, cholesterol, urea, antigen or antibody. The regent system is a porous membrane or bibulous film having a porosity gradient from one planar surface to the other. The sample is applied to the dense side of the membrane which prevents cells in biological samples from entering the membrane having the reagent system.

U.S. Patent No. 4,738,823 describes a test strip with an adjustable sample absorption capacity which can be preselected.

E.P.O. Patent Application No. 0 256 851 (published February 24, 1988) describes a method for determining the presence of an analyte along with an apparatus specifically designed to carry out the method. The method involves taking a reflectance reading from one surface of an inert porous matrix impregnated with a reagent that interacts with the analyte to produce a light-absorbing reaction product when the sample is applied to another surface and migrates through the matrix to the surface being read. Although the method does not involve calculation of a reaction rate, readings are made at two wavelengths to eliminate interference, particularly from blood cells in the sample. The dye couple MBTH-DMAB is used with glucose measuring reagents.

The present invention provides a method for determining the concentration of an analyte in a liquid sample comprising applying the sample to the upper surface of a reagent matrix layer which is asymmetrically porous having pores of progressively decreasing diameter from the upper surface to the lower surface of the reagent matrix layer and determining the amount of analyte from the lower side of the reagent matrix layer. Further the use of the asymmetrically porous reagent matrix layer in a test device is provided. An example of a suitable device comprises three layers: an absorbent layer, a waterproof barrier layer and a reagent matrix layer having a defined saturation volume. The layers in the device are arranged such that the lower surface of the absorbent layer contacts the upper surface of the barrier layer and the lower surface of the barrier layer contacts the upper surface of the reagent matrix layer. Optionally, a support layer contacts the lower surface of the reagent matrix layer. The absorbent layer and barrier layer include an aperture which extends through the absorbent layer and barrier layer permitting application of the sample onto the upper surface of the reagent matrix layer. The reagent matrix layer contains reagent means for producing an amount of a detectable species correlating with the concentration of the analyte in the reagent matrix layer. The absorbent layer absorbs liquid in contact with the reagent matrix layer and absorbent layer sufficiently slowly to permit saturation of the reagent matrix layer. The barrier layer prevents contact of liquid in the absorbent layer with liquid in the reagent matrix layer.

In a preferred embodiment the absorbent layer comprises a hydrophilic paper, the barrier layer comprises a polymer film coated with adhesive on its upper and lower surfaces, and the reagent matrix layer comprises an asymmetrically porous membrane having progressively smaller pores from the upper surface of the reagent matrix layer to the lower surface of the reagent matrix layer. In a most preferred embodiment, the test device quantitates glucose in a whole blood sample.

**In the drawing:**

Fig. 1 is an exploded view of a preferred embodiment of the test device of this invention.

Fig. 2 is a top view of the device shown in Fig. 1.

Fig. 3 is a cross-sectional view of the device taken along line 3-3 in Fig. 2.

Fig. 4 is a cross-sectional view of another embodiment of the test device of this invention.

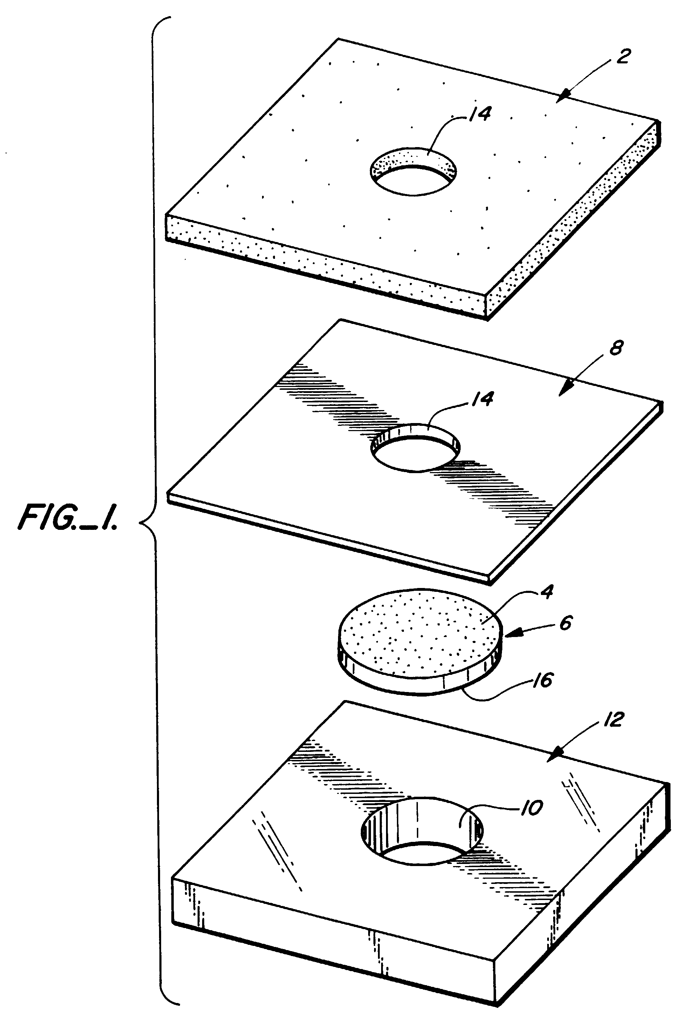

Fig. 1 shows an exploded view of a test device of this invention. The absorbent layer **2** is separated from the dosing surface **4** of the reagent matrix layer **6** by the barrier layer **8**. The optional support layer **12**, in supporting relationship to the reagent matrix layer **6** and the barrier layer **8**, includes an aperture **10** through which the detectable species can be determined at the determination surface, detecting layer surface or determining surface **16**. Aperture **14** extends through each of the absorbent layer **2** and the barrier layer **8** and allows application of a test sample to the dosing surface **4** of the reagent matrix layer **6**.

Fig. 2 is a top view of a device of Fig. 1.

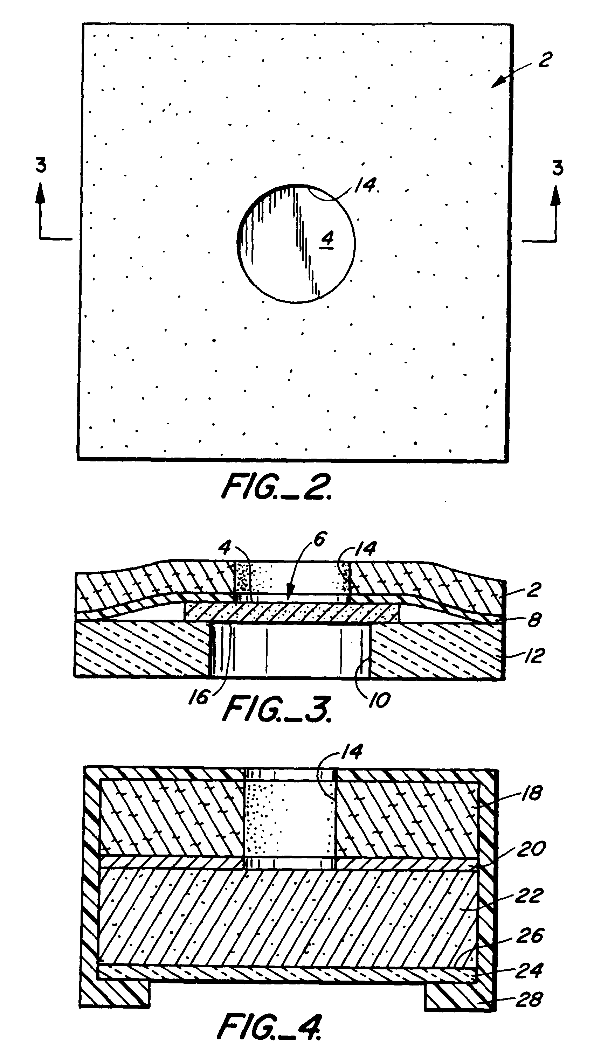

Fig. 3 is a cross-sectional view of a preferred embodiment of a device of the invention taken along line 3-3 in Fig. 2. The absorbent layer **2** and barrier layer **8** detecting layer surface define aperture **14** for application of the test sample onto the dosing surface **4** of the reagent matrix layer **6**, which has a defined, predetermined saturation volume. An opening **10** in the support layer **12** allows visual determination of a detectable species at the determination surface **16** of the reagent matrix layer **6**.

Fig. 4 is a cross-sectional view of an alternate embodiment of the test device wherein the support layer forms an enclosure which holds the absorbent layer **18**, barrier layer **20**, and reagent matrix layer **22** in close proximity. A substantially transparent support layer **24** is held adjacent to the reagent matrix layer **22** at its determination surface **26** by an enclosing support layer **28**. Support layers **24** and **28** may conveniently be manufactured as one unit.

The upper surface of the reagent matrix layer to which the sample is applied is referred to as the dosing surface. The lower surface of the reagent matrix layer where the amount of the detectable species is determined is referred to as the determination surface. Adjacent to the dosing surface of the reagent matrix layer is a barrier layer means. Adjacent to the barrier layer means is an absorbent layer. Each of the barrier and absorbent layer means includes an aperture or hole, for depositing the test sample directly on the dosing surface of the reagent matrix layer.

The reagent matrix layer is quickly absorbent, and sample which is present on its surface quickly saturates the reagent matrix layer. Excess sample remains at the surface of the reagent matrix layer, where it is absorbed more slowly by the absorbent layer. The barrier layer separates the reagent matrix layer from the absorbent layer, so that the two absorbent layers are in fluid contact only while there is unabsorbed sample at the dosing surface of the reagent matrix layer.

The amount of sample placed onto the reagent matrix layer should be in excess of that required to fully saturate the reagent matrix layer. When the reagent matrix layer becomes saturated, further flow of sample into the reagent matrix layer stops. The remainder of the sample is absorbed by the absorbent layer. Excess sample in the absorbent layer remains separated from the reagent matrix layer by the barrier layer and thereby does not affect the reaction.

The method of the present invention are suited to a broad range of volume-sensitive solid phase tests. These include assays for glucose, alcohol, AST (cholesterol), ALT (phenobarbital), theophylline and other chemical assays.

Located at one surface of the reagent matrix layer, and positioned between the reagent matrix layer and the absorbent layer, is the barrier layer. The barrier layer is a waterproof or substantially waterproof material which has at least one aperture. The aperture may be of any shape, for example, square, rectangular, octagonal, ellipsoidal, etc., and is conveniently round. The aperture of the barrier layer may or may not be of similar size, shape, or configuration to that of the absorbent layer. However, the apertures of the two layers must align functionally so that a liquid sample can be inserted conveniently through the openings in the absorbent and barrier layers, and onto the exposed surface of the reagent matrix layer. Conveniently, the aperture is at least 0,127 cm (0.050 inches) in diameter, more generally at least 0,381 cm (0.150 inches), and preferably from 0,457 cm (0.180) to 1,27 cm (0.500 inches).

The barrier layer may be made from any convenient waterproof material, especially in the form of a sheet or foil. Metal sheets or foils include e.g., aluminum foil. Polymeric materials suitable for use include polyethylene, polypropylene, poly(vinyl chloride), cellulose acetates, polyethyleneterephthalates, polycarbonates, or polystyrenes. Various types of paper including paraffin-impregnated paper, polymer laminated paper, water-resistant paper, and wax paper are appropriate, as is glass. The barrier layer may or may not be composed of the same material as the support layer, if any. Alternatively, a solution of a waterproofing material which will form the barrier layer may be coated onto the absorbent layer. A suitable thickness of the waterproof barrier layer is about 3 µm to about 1 mm or less, and more preferably about 10 µm to about 0.4 mm.

Pressure-sensitive, thermosensitive, solvent-based, and reactive adhesives may be used as an adhesive to fix the barrier layer to the absorbent layer, the reagent matrix layer, and/or the support layer. Preferably, a double-sided adhesive tape is utilized to provide the waterproof barrier layer and to provide ease of assembly of the test device.

The absorbent layer is preferably a hydrophilic paper, but other absorbent materials can also be used. Open-pored synthetic resin foams, blush polymers, liquid-resistant gels, fabrics, felts, and the like may be used. Inorganic materials, for example, gypsum, are less preferred since they normally do not possess sufficient stability. The absorbency of these materials should be such that, upon contact of a test sample with the surface of the reagent matrix layer, the edge of the absorbent layer is moistened, but the test sample is substantially absorbed by the reagent matrix layer until the reagent matrix layer is saturated. The absorbent layer will absorb the sample more slowly than the reagent matrix layer. Reagent matrix layer saturation should be achieved in about one-half second to two seconds or less. Excess sample which remains on the surface of the reagent matrix layer should be absorbed by the absorbent layer within 0.5 to 60 seconds, preferably within 1 to 30 seconds, more preferably 1 to 5 seconds.

The absorbent layer is designed so that an excess volume, for example twice, preferably 10 times or greater, of the suggested load for saturation of the reagent matrix layer may be absorbed by the absorbent layer. Removal of excess sample from the dosing surface prevents additional analyte in the sample from entering the reagent matrix layer and participating in the quantitation reaction and also prevents the detectable species formed by the quantitation reaction from diffusing out of the reagent matrix layer. Test devices may be designed to accept a wide variety of sample loads, and specific designs will be apparent to those skilled in the art in view of the teachings herein.

The absorbent layer includes at least one aperture through which a sample may be deposited on the dosing surface of reagent matrix layer. This aperture should generally coincide with the aperture in the barrier layer so that a functional unit is formed. However, the apertures in each of the absorbent and barrier layers need not be of identical size or location relative to each other to be functional.

In a preferred embodiment, an aperture is provided through the support layer to allow exposure of the determination surface **16** of the reagent matrix layer **6** to the atmosphere, and to permit visual or spectrophotometric determination of the detectable species. Analyses which are preferably performed while exposed to atmospheric oxygen are easily accommodated by minimizing support layer surfaces and maximizing the surface area of the reagent matrix layer which is exposed to the atmosphere.

The nature of the support layer is not particularly limited, as long as it is impermeable to liquid, and can transmit light or other means necessary for determining the detectable species. For example, various polymeric materials, such as polyethylene terephthalates, polycarbonates or polystyrenes, or materials such as glass, or wax paper are suitable for this purpose. The support layer employed may have any desired thickness, but generally a thickness of about 50 µm to about 2 millimeters is adequate. The reagent matrix layer may be directly coated on the support layer. When the reagent matrix layer is a sponge-like material, it may be desirable to glue or otherwise laminate or affix the reagent matrix layer to the support layer. Alternatively, the support layer may form an enclosure which holds the reagent matrix layer, barrier layer, and absorbent layer in close proximity.

Suitable support layers may be opaque or transparent. If no aperture through the support layer is provided, it is essential that the support layer be substantially transparent, so that determination of the detectable species may be made through the support layer and be permeable to air.

In an alternate embodiment of the test device, no support layer, carrier or holder is provided, and the test device consists of a reagent matrix layer, barrier layer, and absorbent layer laminated together in that order. Such devices may be placed on any convenient surface when the assay is conducted. In another embodiment, the reagent matrix layer is enclosed within a support layer that serves as a holder, such that only the surface area which is coincident with the aperture in the absorbent and barrier layers is exposed. Such a configuration is shown in Fig. 4.

The reagent matrix layer is a quickly absorptive, hydrophilic region which performs three functions: (1) it provides a determinate volume for reagents and the test sample; (2) it filters cellular components of the test sample such that cellular components are retained at, or close to, the surface of the layer, while the non-cellular, liquid component of the sample is transported throughout the reagent matrix layer; and (3) it provides a sensing region for determination of the presence of detectable species which minimizes background interference from red blood cells and other cellular components of the test sample.

The reagent matrix layer can be a single membrane or may be formed of a plurality of layers which perform the desired functions and are laminated together. The upper portion of the reagent matrix layer which includes the dosing surface of reagent matrix layer contains pores which filters out or entrap particulate matter in the sample, particularly red blood cells. The lower portion of the reagent matrix layer provides a region having an appropriate color to facilitate determining the detectable species.

The reagent matrix layer provides a defined space which absorbs a predetermined volume of the sample. Additionally, the reagent matrix layer is substantially or completely opaque to prevent interference with the detectable species by red blood cells. A membrane which is opaque to red blood cells and is porous, permitting the liquid portion of the sample to saturate the reagent matrix layer can be included, if the reagent matrix layer is not substantially opaque. Preferably the opaque membrane will be in the upper portion of the reagent matrix layer, but below the region where the red blood cells are trapped.

Materials which are appropriate for use as the base portion of the reagent matrix layer include glass fibers and foam filters of the prior art. The lower surface of the reagent matrix layer provides a region where the detectable species is determined with minimal interference by red blood cell pigmentation. The lower portion can be comprised of the same materials as the upper portion.

The reagent matrix layer comprises a porous member which is asymmetrically porous, having pores of progressively decreasing diameter in a progression from the upper (dosing) surface to the lower (determination) surface of the reagent matrix layer. A blown-pore or open-pore structure such as that found in the BTS Asymmetric membrane from Filtrite (San Diego, CA) or that shown by U.S. Patent 3,158,532, issued to Pall et al is especially preferred and can serve as the reagent matrix layer without the use of additional membranes or layers. Such membranes have the advantage of separating cellular blood components. When whole blood is applied to the test device, red blood cells are localized at the dosing surface. Serum blood components progress throughout the porous medium, and the reagent-analyte reaction occurs throughout the matrix.

Additionally, the surface to volume ratio of the asymmetrical membranes is very large, providing rapid absorption of the sample. Rapid, uniform wetting of the reagent matrix layer dissolves the dried reagents and places the analyte in close proximity with the reagents, reducing the amount of time necessary to reach the endpoint of the reaction. Rapid, uniform wetting of the reagent matrix layer allows a determination which is not substantially affected by the presence of excess sample on the dosing surface of the reagent matrix layer.

It is an advantage of this invention that the volume of the reagent matrix layer upon sample saturation may be precisely calculated, as any excess sample is either absorbed by the absorbent layer or assayed so rapidly that no interference with the quantitation reaction is observed. In this way, the amount of analyte in the sample can be calculated based on a single determination that indicates the amount of reaction with sample analyte, rather than on two determinations which are used to calculate the rate of the reaction. Additionally, the exact amount of reagent necessary for quantitative analysis of the analyte in the test sample may be calculated and provided within the reagent matrix layer.

In use, the amount of excess sample applied to the exposed surface of the matrix becomes unimportant, within wide tolerances. Non-cellular components of the sample placed on the dosing surface of the reagent matrix layer pass into the layer until the reagent matrix layer is saturated. Upon saturation, the excess sample remains on top of the layer, where it is absorbed by the absorbent layer and is prevented from reacting with reagents in the reagent matrix layer by the barrier layer. Accordingly, the test device achieves defined loading of sample per unit regardless of the amount of sample applied to the test device and a defined volume test device is obtained. A device is easily designed so that only a fraction of a standard drop of sample is necessary to fully saturate the reagent matrix layer.

In a preferred embodiment, the reagent matrix layer includes a single membrane or filter medium which exhibits progressively finer filtration in a progression from the dosing surface to the lower surface, so that cellular components of the test sample are localized at the upper surface of the membrane. This medium should be opaque, and have light or dark color characteristics. When the detectable species is a dye, it is preferable to have an opaque, light-colored or white background, for color detection.

The reagent matrix layer includes one or more chemical reagents which are capable of directly or indirectly reacting with the analyte of interest to produce a detectable species. Reagents which give rise to a colored reaction product or cause a distinct change in color are preferred for ease of reading. Reagents which provide chemiluminescence, or other detectable products, are also appropriate for use. Specialized apparatus for determining the extent of reaction may be necessary for non-visually determinable products.

The substances in blood, serum, urine, cerebrospinal fluid, lymph, or other bodily fluid, which can be measured by the test device of this invention include glucose, galactose, pyruvic acid, amino acids, cholesterol lactic acid, alcohol, urea, etc. The reagents used for measurement vary depending on the analyte to be measured and do not differ from those used in other multilayer pad analysis devices.

The detectable species is fully formed, and the assay is ready for determination of the detectable species after 1 minute or less, preferably 30 to 45 seconds, more preferably less than 30 seconds. That is, when the analyte is in the average or normal value range, the endpoint of the assay is reached in 1 minute or less. For samples with a high analyte concentration, the endpoint is preferably reached in less than two minutes, preferably, in one minute or less. Alternatively, a plurality of readings can be made to determine whether the endpoint had been reached, especially for concentrations in the high end of the normal range. The endpoint can be determined by making readings until there is not further change or by performing simple calculations based on two or more readings when the reaction is substantially complete, i.e. about 70 to 90% complete.

For example, a test device for measuring glucose in blood may contain glucose oxidase, a substance having peroxidase activity, and an oxidizable indicator as ingredients. Similarly, a test device for measuring galactose in blood may contain galactose oxidase, a substance having peroxidase activity, and an oxidizable indicator. Where the alcohol level in blood is to be measured, a test device may be impregnated with a reagent system comprising alcohol dehydrogenase, nicotine adenine dinucleotide, diaphorase and a tetrazolium salt.

The detectable species of the reagent matrix layer may be varied to facilitate the detection process. The terms "detectable chemical species" and "detectable species" refer to a chemical species which provides a detectable signal or change that is directly or indirectly related to the concentration in the reagent matrix layer of a desired analyte, or a reaction or decomposition product of the analyte, e.g., the optical density of color formed, fluorometric density, electromagnetic (including radiation) intensity or a change in these densities or intensities. Preferably, the detectable species is optically detectable, e.g., visually detectable, or detectable using spectrophotometry.

The preferred dyes or other detectable chemical species will differ for various assays, and are well known to those skilled in the art. Exemplary of dyes which are appropriate for use in an assay for glucose in a body fluid are 3,5-dimethylaminobenzoic acid (DMAB); 3-methyl-2-benzothiazolinone hydrazone hydrochloride (MBTH); and/or tetramethyl benzadine (TMB).

A preferred dye is DMAB, which shows an absorption peak at 570-600 nm. Although the dye is visually detectable, a device such as a spectrophotometer is preferably used to determine the dye produced. In particular, it has now been found that when reflectance is determined at a wavelength greater than the dye peak, the effect of red blood cells is minimized, particularly at low glucose concentrations. The dye is determined at 600-620nm, preferably at about 610nm. This preferred dye and determination wavelength act to minimize interference when red blood cells are present at the dosing surface of the reagent matrix layer, as described in detail in Example 7.

In most cases, the means for measuring a specific analyte is not limited to one set of reagents, and a plurality of suitable reagents are known. Any chemical method may be used so long as the reagent are stable in the reagent matrix layer. Further, one may add a substance to buffer the reagent composition to a constant pH, a substance to stabilize it, a hydrophilic substance to aid absorption of the test sample into the reagent matrix layer, etc. Such modifications are well known by those skilled in the art.

Suitable buffering substances include physiologic buffers that maintain the pH between 6.5 and 8.5, preferably between 7 and 8, such as Tris buffer, HEPES buffer and the like. Exemplary stabilizers include proteinaceous substances such as bovine serum albumin and caesin, acacia, PVP and EDTA. Hydrophilic substances that aid in absorption of the test sample include surfactants, preferably fluorinated surfactants.

To prepare the reagent matrix layer, the reagents and additives are dissolved in water to produce an aqueous solution. The reagent matrix layer is dipped into the solution and dried. The reagent matrix layer may be squeezed prior to drying, to hasten the drying time. When water-insoluble or substantially water-insoluble reagents are used, the reagents are dissolved in an appropriate solvent and the reagent matrix layer is dipped into the solvent either following or, preferably, prior to the aqueous solution dip and drying step. To be suitable, a solvent must dissolve the reagents and the reagent matrix layer must be stable in the solvent. Reagents such as.cellulose acetate, which facilitates uniform wetting of the reagent matrix layer and control of pore size, are insoluble in water but are soluble in glacial acetic acid and a mixture of glacial acetic acid and isopropanol. When using the dye couple DMAB and MBTH, DMAB is preferably dissolved in the non-aqueous solvent to prevent reaction of DMAB and MBTH in the solution. The coated reagent matrix layer may be cut into smaller units, or otherwise processed to prepare the test devices of this invention.

To prepare the device, the openings in the covering absorbent and barrier layers may be formed either before or after (but normally before) the absorbent and barrier layers have been applied or joined to the reagent matrix layer. The layers may be laminated together, applied by chemical deposition, or contained in an enclosing holder. In an especially preferred embodiment, the barrier layer comprises double-sided tape that laminates the reagent matrix layer, the absorbent layer, and the support layer together.

The general structure and function of elements of the test device have been described. For purposes of illustration, the device, and methods of making and using the device will be described for a glucose assay.

In an assay for glucose, an enzyme system having glucose oxidase activity is provided, together with a substance having peroxidase activity and a substance which undergoes a color change upon reaction with one or more of the compounds formed during the action of the enzymes upon glucose-containing fluids. Such reagent systems are well known and commercially available.

To measure glucose, a drop of blood drop from the earlobe, finger tip, or other source, is applied to the dosing surface of the reagent matrix layer. Glucose in the blood sample is converted to gluconic acid by glucose oxidase. Hydrogen, released by this reaction, combines enzymatically with atmospheric oxygen to form hydrogen peroxide. In the presence of a peroxidase, hydrogen peroxide oxidizes the indicator, producing a detectable species. Generally, the detectable species is formed in two minutes or less, preferably three seconds or less, from the time of dosing of the test sample. When the detectable species is determined visually, the concentration of glucose is calculated by comparing the color produced with a standard color chart which is prepared separately.

When an electromechanical device is to be used to determine the glucose concentration optically, the reflectance of the color produced is measured at a predetermined wavelength by means of an appropriate detector such as a light detection diode. Preferably, the determination surface will be illuminated by a light source a predetermined, specific wavelength The concentration of glucose is determined with reference to a concentration-reflectance standard values which are separately determined. Preparation and use of an exemplary glucose test device is described in detail in the Examples.

A device to be used in an assay for alcohol in a test sample comprises, sequentially, an absorbent layer, a barrier layer, and a reagent matrix layer including a chemical system which produces a detectable species in the presence of alcohol, such as alcohol oxidase, peroxidase, and a suitable dye. Apertures through the absorbent and barrier layers are provided. A test sample, such as a drop of whole blood, serum, urine, or other bodily fluid, is placed through the apertures and onto the dosing surface of the reagent matrix layer. The presence and extent of the detectable species is determined, as appropriate to the detectable species used. When the detectable species is a dye such as tetramethyl benzadine (TMB), the determination may be made visually, with a spectrophotometer or with a light detecting diode or other light sensor.

The alcohol assay device produces a quick, quantitative assay without the need to accurately measure the sample provided to the dosing surface. A small sample, e.g., a drop of whole blood, is sufficient to provide results. The device may be made as a disposable unit. When a colorfast dye is used, the device may be retained as evidence of the assay. The device is not particularly temperature sensitive, and may be used in a variety of field conditions, e.g., roadside checkpoints for alcohol use while operating a motor vehicle. Except for the enzymes, the device need not differ from a glucose test device. The device may also include a substance which stabilizes alcohol, such as sugars, e.g. mannitol.

A device to be used in an assay for cholesterol in a test sample according to this invention comprises, sequentially, an absorbent layer, a barrier layer, and a reagent matrix layer including a chemical system which produces a detectable species in the presence of cholesterol, such as cholesterol esterase, cholesterol oxidase, and peroxidase with a suitable dye. Apertures through the absorbent and barrier layers are provided. A test sample, such as a drop of whole blood, serum, or plasma, is placed through the apertures and onto the dosing surface of the reagent matrix layer. The presence and extent of the detectable species is determined, as appropriate to the detectable species used. When the detectable species is a dye such as TMB, the determination may be made visually, or with a spectrophotometer.

The device may be used for continued monitoring of a patient's cholesterol levels in a disposable home test. The cholesterol assay device produces a quick, quantitative assay without the need to accurately measure the sample provided to the dosing surface. A small sample, i.e., a drop of blood, is sufficient. Instructions are simple, and only minimal training is required for accurate use of the device. The device can be similar to the glucose or alcohol test devices except that the enzymes differ. Additionally, a substance which solubilizes cholesterol will be included in the device. Useful solubilizing agents include surfactants, preferably methylglucamides such as MEGA 8 (Behring, La Jolla, CA)

This invention is further illustrated by the following specific, but non-limiting examples. Temperatures are given in degrees Centigrade and percents as weight percents unless otherwise specified. Examples which are constructively reduced to practice herein are presented in the present tense, and examples representing laboratory experiments previously reduced to practice are presented in the past tense.

### EXAMPLE 1

### Preparation of Glucose Reagent Matrix Layer

Solutions A and B were prepared according to the following procedure.

### Solution A:

In a fume hood, measure 600 mL glacial acetic acid into a container. Weigh 6.0 grams of cellulose acetate. Slowly add the cellulose acetate to the glacial acetic acid with constant stirring. Avoid forming clumps. Stir until dissolved. Weigh and add 36 g DMAB. Stir until dissolved.

Measure 600 mL isopropanol; add dropwise to the stirring solution. If added too fast, the cellulose acetate will precipitate. A localized haze at the site of isopropanol addition indicates either too rapid an addition or insufficient mixing.

Add 1.2 mL FC-129® surfactant (3M, St. Paul, MN). Mix for 3 to 7 minutes.

### Solution B:

### Diluent:

Dry acacia and PVP K-30® (GAF, NY, NY) by heating at approximately 90°C for at least 4 hours before use. Cool in a desiccator before weighing.

Add approximately 0.8 L distilled water to a container which has a stir bar. Weigh and add 2.75 g EDTA disodium: stir until dissolved. Weigh and add 10.56 g citric acid and 13.32 g Tris (0.1 M). Stir until dissolved.

Weigh 33.00 g of the dried acacia, and slowly add it to the solution. Avoid forming clumps, which will take longer to dissolve. Stir until dissolved.

Weigh 33.00 g of the dried PVP K-30®, and add to the solution with stirring, until dissolved.

Adjust the pH to 5.0±0.1 with 5N NaOH (as required, approximately 3 mL).

Add distilled water to bring the final volume to one liter. Check the pH. Filter the solution through a Sartorius 0.45 µ filter capsule.

### Enzyme Solution:

Measure 909 mL Solution B Diluent into a container; allow to come to room temperature.

Measure 90.9 mL distilled water into a container. Add 500,000 U horseradish peroxidase and 500,000 U glucose oxidase to the distilled water; mix until dissolved.

Add 8 g MBTH to Solution B Diluent; stir until dissolved.

Mix the enzyme/distilled water solution into the Solution B Diluent/MBTH solution. Transfer the solution several times from one container to the other to insure that the enzyme solution is rinsed from the container. Mix gently for 5-15 minutes, until the solution is homogeneous.

An asymmetric polysulfone membrane (BTS™ filter, Filtrite, San Diego, CA) was dipped in Solution A. The membrane was gently squeezed between rollers to remove excess solution and to aid drying and air dried until the solvent has evaporated. The dried membrane was then dipped into Solution B, gently squeezed between rollers to remove excess solution and to aid drying, and air dried until the solvent has evaporated. Drying times are approximately ten minutes at 50°C.

### EXAMPLE 2

### Assembly of Test Device

Tape having 415 adhesive from 3M was cut into 1/2 inch strips and adhered to an absorbent paper (Whatman #3 paper, Whatman, England). A circular opening approximately 0.187 inches in diameter was made through the tape and absorbent layers at 1 inch intervals along the tape. A reagent matrix layer according to Example 1 which had been trimmed to a 0.250 inch diameter was aligned with the circular opening, and applied to the exposed adhesive surface. A plastic strip 1/2 inch wide and having a series of circular openings approximately 0.187 inches in diameter at 1 inch intervals along the plastic strip was aligned with and laminated to the adhesive tape/membrane strip over the reagent matrix layer. The strip was then cut into 1 inch segments, each segment containing a sample application aperture and a dye determination aperture in the center of opposing sides. The saturation volume of the reagent matrix layer is approximately 3 µL.

### EXAMPLE 3

### Glucose Testing of Blood Sample

A drop of blood from a finger puncture was placed on to the test device of Example 2 at the opening through the barrier layer. The test device was allowed to stand for at least one second, preferably one minute. The detectable species was determined using a spectrophotometer at 610 nm. The spectrophotometric result was compared to a standard curve to determine the quantity of glucose in the blood sample.

### EXAMPLE 4

### Alcohol Detection Reagent Matrix Layer

An alcohol detection membrane is formed by the process of Example 1, substituting 30,000-50,000 U of alcohol oxidase for the glucose oxidase of Solution B.

A test device using the alcohol detection membrane is manufactured according to the process of Example 2, substituting the alcohol detection membrane for that of Example 1. A drop of whole blood is placed onto the test device at the dosing surface. The dye production is evaluated at the determination surface using a spectrophotometer at 610 nm, and compared to a standard, to ascertain the amount of alcohol in the blood sample.

### EXAMPLE 5

### Cholesterol Detection Reagent Matrix Layer

A cholesterol detection membrane is formed by the process of Example 1, substituting 100-200 U cholesterol esterase and 100-200 U cholesterol oxidase for the glucose oxidase of Solution B, and 1000 U peroxidase is substituted for the peroxidase of Solution B.

A test device is manufactured according to the process of Example 2, substituting the cholesterol detection membrane for that of Example 1. A drop of whole blood is placed onto the test device at the exposed dosing surface. The dye production is visually evaluated at the determination surface and compared to a standard, to ascertain the amount of cholesterol in the blood sample.

### EXAMPLE 6

### Glucose Testing of a Urine Sample

A test device according to Example 2 is constructed. A drop of urine is placed onto the exposed dosing surface of the test device. The test device is allowed to stand at least one second. The color is determined, and compared to a standard curve to determine the quantity of glucose in the urine sample.

### EXAMPLE 7

### Hematocrit Effects

It has been unexpectedly discovered that the signal-to-noise ratio was maximized, and the variable hematocrit effect minimized, when a reflectance determination was made using light having a wavelength of 610 nm.

Calculated glucose was determined using an assay device prepared according to Example 2. Actual glucose values were determined using the YSI Model 21 instrument (Yellow Springs Instruments, Yellow Springs, OH) according to the manufacturer's directions.

The ratio calculated glucose to actual glucose is shown in Table A. In Table A, "Calc.Glu." indicates the amount of glucose as determined by a device of this invention; "Std.Glu." indicates the actual amount of glucose by the standard determination method; and "Calc./Std." indicates the ratio of the glucose concentration value as determined by a device of this invention as compared to the standard determination. A ratio of 1.0 reflects agreement between both methods of determination. A ratio of less than 1.0 reflects that the actual glucose value was under-represented by a device of this invention. A ratio of greater than 1.0 reflects over-representation of the glucose value.

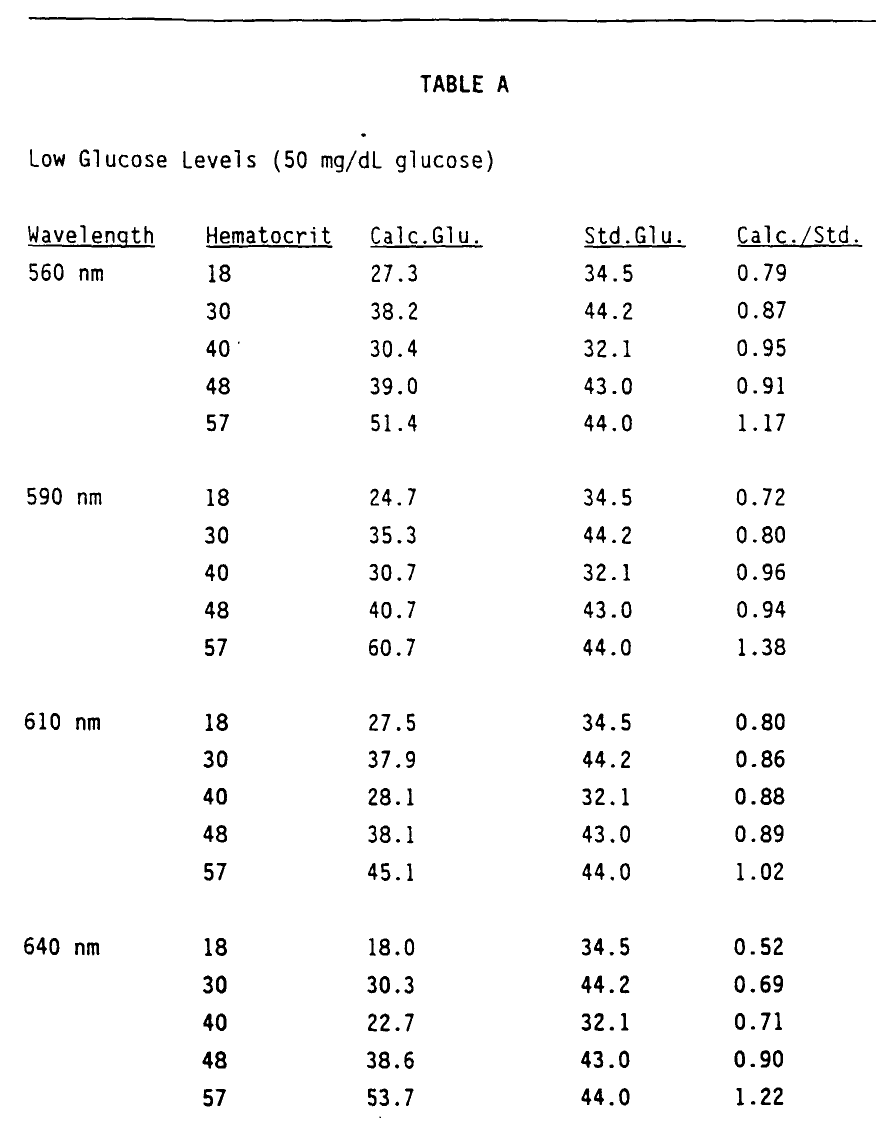

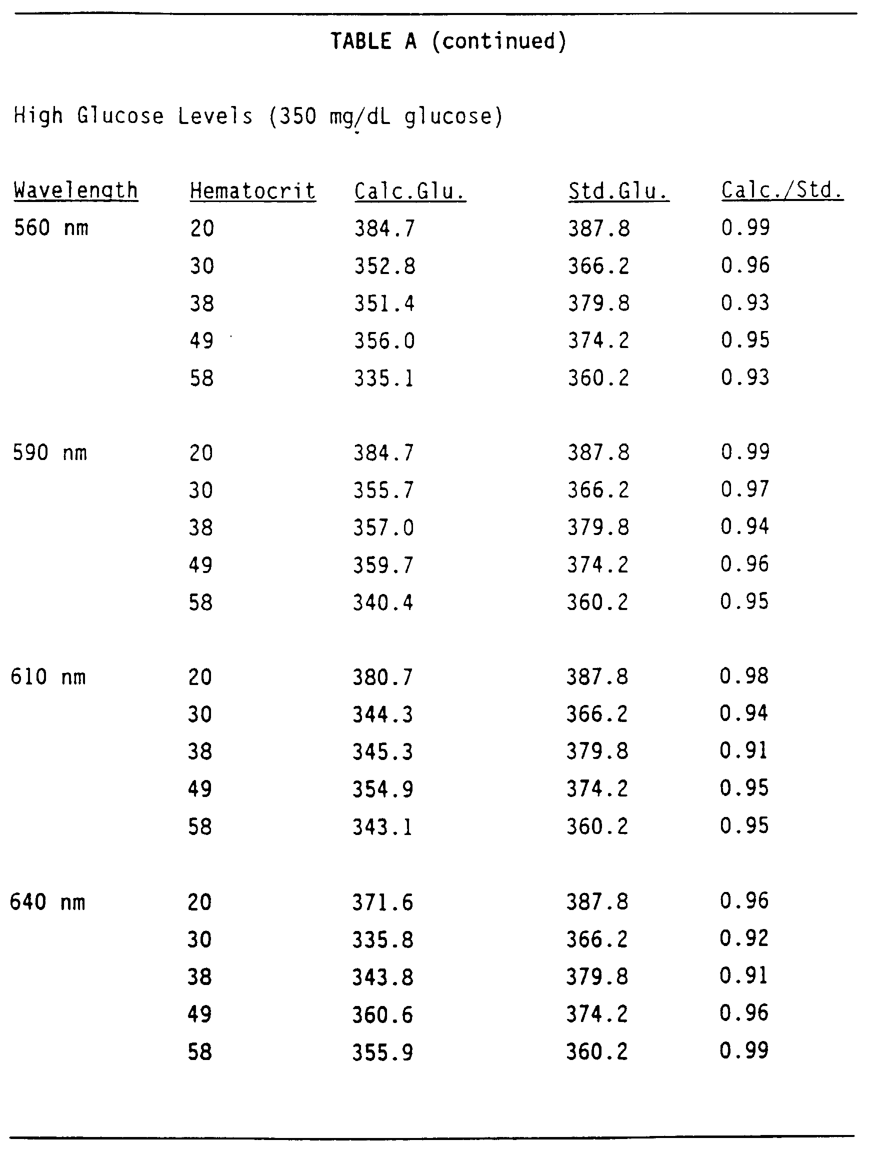

As shown in the table, at low glucose concentrations and low hematocrit levels, glucose tends to be under-represented. At low glucose levels and high hematocrit levels, the glucose tends to be over-represented. Surprisingly, this variation was minimized when reflectance was determined at about 610 nm.

The range of variation at high glucose levels was substantially less than that at low glucose levels, at all hematocrit levels.

Determination of the dye species at about 610 nm minimized variation for both high and low glucose concentrations, at all hematocrit levels.

## Claims

1. A method for determining the amount of an analyte in a liquid sample compnsing applying the sample to the upper surface of reagent matrix layer which provides a determinate volume for sample and test reagents and is asymmetrically porous, having pores of progressively decreasing diameter from the upper surface to the lower surface of the reagent matrix layer and determining the amount of analyte from the lower surface of the reagent matrix layer.

2. The method of claim 1 wherein excess sample liquid remaining on the upper surface of the reagent matrix layer after the reagent matrix layer is saturated with liquid sample and being in contact with the absorbent layer is absorbed by an absorbent layer.

3. The method of claims 1 or 2 wherein the reagent matrix layer includes 3,5-dimethylaminobenzoic acid, 3-methyl-2-benzothiazoline hydrazone hydrochloride, glucose oxidase and peroxidase.

4. Use of an asymmetrically, porous membrane providing a determinate volume for sample and test reagents and having progressively smaller pores from the upper surface to the lower surface as a reagent matrix layer in a test device in such a way that a sample is applied to the large pore surface.

## Patentansprüche

1. Verfahren zur Bestimmung der Menge eines Analyten in einer flüssigen Probe, umfassend das Aufbringen der Probe auf die obere Fläche einer Reagensmatrixschicht, die ein festgelegtes Volumen für die Probe und die Testreagenzien bereitstellt und asymmetrisch porös ist und Poren aufweist, deren Durchmesser von der oberen Fläche zur unteren Fläche der Reagensmatrixschicht hin immer kleiner wird, und das Bestimmen der Analytmenge von der unteren Fläche der Reagensmatrixschicht aus.

2. Verfahren nach Anspruch 1, wobei überschüssige Probenflüssigkeit, die auf der oberen Fläche der Reagensmatrixschicht verbleibt, nachdem die Reagensmatrixschicht mit flüssiger Probe gesättigt ist, und in Kontakt mit der absorbierenden Schicht steht, durch eine absorbierende Schicht absorbiert wird.

3. Verfahren nach Anspruch 1 oder 2, wobei die Reagensmatrixschicht 3,5-Dimethylaminobenzoesäure, 3-Methyl-2-benzothiazolonhydrazon-hydrochlorid, Glucoseoxidase und Peroxidase enthält.

4. Verwendung einer asymmetrisch porösen Membran, die ein festgelegtes Volumen für die Probe und die Testreagenzien bereitstellt und Poren aufweist, die von der oberen Fläche zur unteren Fläche hin immer kleiner werden, als Reagensmatrixschicht in einer Testvorrichtung in einer Weise, daß eine Probe auf die Fläche mit den großen Poren aufgebracht wird.

## Revendications

1. Procédé pour déterminer la quantité d'un analyte dans un échantillon liquide consistant à appliquer l'échantillon à la surface supérieure d'une couche de matrice réactive qui fournit un volume déterminé pour l'échantillon et les réactifs d'essai et qui est asymétriquement poreux, ayant des pores de diamètre progressivement décroissant à partir de la surface supérieure vers la surface inférieure de la couche de matrice réactive et à déterminer la quantité d'analyte à partir de la surface inférieure de la couche de matrice réactive.

2. Procédé selon la revendication 1, dans lequel le liquide échantillon en excès restant sur la surface supérieure de la couche de matrice réactive, après que la couche de matrice réactive est saturée avec l'échantillon liquide et étant en contact avec la couche absorbante, est absorbé par une couche absorbante.

3. Procédé selon la revendication 1 ou 2, dans lequel la couche de matrice réactive inclut l'acide 3,5-diméthylaminobenzoïque, le chlorhydrate de la 3-méthyl-2-benzothiazolinonehydrazone, la glucose oxydase et la peroxydase.

4. Utilisation d'une membrane asymétriquement poreuse fournissant un volume déterminé pour l'échantillon et les réactifs d'essai et ayant des pores progressivement plus petits à partir de la surface supérieure vers la surface inférieure en tant que couche de matrice réactive dans un dispositif d'essai d'une façon telle qu'un échantillon est appliqué à la grande surface de pores.
